# EUROPEAN PATENT APPLICATION

(11) **EP 4 600 238 A1**
(43) Date of publication of application: **13.08.2025**
(21) Application number: 23874925.3
(22) Date of filing: 04.10.2023
(51) Int. Cl.: C07C 19/16, C07C 31/36, C07C 31/38, C07C 53/21

(54) **FLUORINE-CONTAINING COMPOUND AND PRODUCTION METHOD OF SAME AND SURFACTANT**

(30) Priority: 04.10.2022 JP 2022160510
(71) Applicant: DAIKIN INDUSTRIES, LTD., Osaka-shi, Osaka 530-0001 (JP)
(72) Inventor: KISHIKAWA, Yosuke, Osaka-Shi, Osaka 530-0001 (JP); HOSHIYA, Naoyuki, Osaka-Shi, Osaka 530-0001 (JP); ISHIHARA, Sumi, Osaka-Shi, Osaka 530-0001 (JP)
(74) Representative: Hoffmann Eitle
(86) International application number: PCT/JP2023/036296
(87) International publication number: WO 2024/075800

(57) **Abstract**

A compound represented by general formula: R¹-R²-X, wherein R¹ is -CH₃, -CH₂F, -CHF₂, -CH₂I, -CHFI, or an anionic group, R² is an alkylene group consisting of unit represented by -CFH-, or an alkylene group consisting of unit represented by -CFH- and a unit represented by -CH₂-, provided that these alkylene groups optionally contain epoxy group, -CH(OH)-, - CHI-, or a divalent cycloalkylene group, X is -OH, -CH(R²¹)OH (wherein R²¹ is H, a non-fluorinated alkyl group, or a fluorinated alkyl group), -I, -CFHI, -CH₂I, an anionic group, or -COOR²² (wherein R²² is a non-fluorinated alkyl group having 1 to 8 carbon atoms, and the total number of carbon atoms of R¹, R², and X is 2 to 50).

## Description

### TECHNICAL FIELD

The present disclosure relates to a fluorine-containing compound, a method for producing the same, and a surfactant.

### BACKGROUND ART

Patent Document 1 discloses a compound having a formula Rf-(CH₂)ₘ-R'f-COOY, wherein m is 1 to 3, Rf is a perfluoroalkyl or a perfluoroalkoxy having 3 to 8 carbon atoms, R'f is a linear or branched perfluoroalkylene having 1 to 4 carbon atoms, Y is M or R, M is NH₄, Li, Na, K, or H, and R is a linear, branched, or a cyclic alkyl having 1 to 8 carbon atoms.

Patent Document 2 discloses a fluorosurfactant containing a compound of formula (I):

RfCH₂OCF(CF₃)C(O)OM (I)

wherein Rf is a linear or branched perfluoroalkyl group having 2 to 5 carbon atoms, and M is H, NH₄, Li, Na, or K.

### RELATED ART

### PATENT DOCUMENTS

Patent Document 1: Japanese Patent Laid-Open No. 10-212261
Patent Document 2: National Publication of International Patent Application No. 2012-513531

### SUMMARY OF THE INVENTION

### PROBLEM TO BE SOLVED BY THE INVENTION

However, each of the compounds described in Patent Documents 1 and 2 is a compound having CF₃- (trifluoromethyl group) at a molecular end or a compound containing unit represented by -CF₂- (difluoromethylene group) in the molecule, and no fluorine-containing compounds containing neither CF₃-nor unit represented by -CF₂- are disclosed.

An object of the present disclosure is to provide a novel fluorine-containing compound containing neither trifluoromethyl group nor difluoromethylene group.

### MEANS FOR SOLVING THE PROBLEM

The present disclosure provides a compound represented by general formula: R¹-R²-X wherein R¹ is -CH₃, -CH₂F, -CHF₂, - CH₂I, -CHFI, or an anionic group; R² is an alkylene group consisting of a unit represented by -CFH-, or an alkylene group consisting of unit represented by -CFH- and unit represented by -CH₂-, provided that these alkylene groups optionally contain epoxy group, -CH(OH)-, -CHI-, or a divalent cycloalkylene group; X is -OH, -CH(R²¹)OH (wherein R²¹ is H, a non-fluorinated alkyl group, or a fluorinated alkyl group), - I, -CFHI, -CH₂I, an anionic group, or -COOR²² (wherein R²² is a non-fluorinated alkyl group having 1 to 8 carbon atoms); and the total number of carbon atoms of R¹, R², and X is 2 to 50.

### EFFECTS OF INVENTION

The present disclosure provides a novel fluorine-containing compound containing neither trifluoromethyl group nor difluoromethylene group.

### DESCRIPTION OF EMBODIMENTS

Before describing the present disclosure in detail, some terms used in the present disclosure will now be defined or described.

The "organic group" as used herein means a group containing one or more carbon atoms or a group formed by removing one hydrogen atom from an organic compound.

Examples of the "organic group" include:
an alkyl group optionally having one or more substituents,
an alkenyl group optionally having one or more substituents,
an alkynyl group optionally having one or more substituents,
a cycloalkyl group optionally having one or more substituents,
a cycloalkenyl group optionally having one or more substituents,
a cycloalkadienyl group optionally having one or more substituents,
an aryl group optionally having one or more substituents,
an aralkyl group optionally having one or more substituents,
a non-aromatic heterocyclic group optionally having one or more substituents,
a heteroaryl group optionally having one or more substituents,
a cyano group,
a formyl group,
RaO-,
RaCO-,
RaSO₂-,
RaCOO-,
RaNRaCO-,
RaCONRa-,
RaOCO-,
RaOSO₂-, and
RaNRbSO₂-
wherein each Ra is independently
an alkyl group optionally having one or more substituents,
an alkenyl group optionally having one or more substituents,
an alkynyl group optionally having one or more substituents,
a cycloalkyl group optionally having one or more substituents,
a cycloalkenyl group optionally having one or more substituents,
a cycloalkadienyl group optionally having one or more substituents,
an aryl group optionally having one or more substituents,
an aralkyl group optionally having one or more substituents,
a non-aromatic heterocyclic group optionally having one or more substituents, or
a heteroaryl group optionally having one or more substituents; and
Rb is independently H or an alkyl group optionally having one or more substituents.

The organic group is preferably an alkyl group optionally having one or more substituents.

The "substituent" as used herein means a group capable of replacing another atom or group. Examples of the "substituent" include an aliphatic group, an aromatic group, a heterocyclic group, an acyl group, an acyloxy group, an acylamino group, an aliphatic oxy group, an aromatic oxy group, a heterocyclic oxy group, an aliphatic oxycarbonyl group, an aromatic oxycarbonyl group, a heterocyclic oxycarbonyl group, a carbamoyl group, an aliphatic sulfonyl group, an aromatic sulfonyl group, a heterocyclic sulfonyl group, an aliphatic sulfonyloxy group, an aromatic sulfonyloxy group, a heterocyclic sulfonyloxy group, a sulfamoyl group, an aliphatic sulfonamide group, an aromatic sulfonamide group, a heterocyclic sulfonamide group, an amino group, an aliphatic amino group, an aromatic amino group, a heterocyclic amino group, an aliphatic oxycarbonylamino group, an aromatic oxycarbonylamino group, a heterocyclic oxycarbonylamino group, an aliphatic sulfinyl group, an aromatic sulfinyl group, an aliphatic thio group, an aromatic thio group, a hydroxy group, a cyano group, a sulfo group, a carboxy group, an aliphatic oxyamino group, an aromatic oxy amino group, a carbamoylamino group, a sulfamoylamino group, a halogen atom, a sulfamoylcarbamoyl group, a carbamoyl sulfamoyl group, a dialiphatic oxyphosphinyl group, and a diaromatic oxyphosphinyl group.

The aliphatic group may be saturated or unsaturated, and may have a hydroxyl group, an aliphatic oxy group, a carbamoyl group, an aliphatic oxycarbonyl group, an aliphatic thio group, an amino group, an aliphatic amino group, an acylamino group, a carbamoylamino group, or the like. Examples of the aliphatic group include alkyl groups having 1 to 8 and preferably 1 to 4 carbon atoms in total, such as a methyl group, an ethyl group, a vinyl group, a cyclohexyl group, and a carbamoylmethyl group.

The aromatic group may have, for example, a nitro group, a halogen atom, an aliphatic oxy group, a carbamoyl group, an aliphatic oxycarbonyl group, an aliphatic thio group, an amino group, an aliphatic amino group, an acylamino group, a carbamoylamino group, or the like. Examples of the aromatic group include aryl groups having 6 to 12 carbon atoms and preferably 6 to 10 carbon atoms in total, such as a phenyl group, a 4-nitrophenyl group, a 4-acetylaminophenyl group, and a 4-methanesulfonylphenyl group.

The heterocyclic group may have a halogen atom, a hydroxy group, an aliphatic oxy group, a carbamoyl group, an aliphatic oxycarbonyl group, an aliphatic thio group, an amino group, an aliphatic amino group, an acylamino group, a carbamoylamino group, or the like. Examples of the heterocyclic group include 5- or 6-membered heterocyclic groups having 2 to 12 and preferably 2 to 10 carbon atoms in total, such as a 2-tetrahydrofuryl group and a 2-pyrimidyl group.

The acyl group may have an aliphatic carbonyl group, an arylcarbonyl group, a heterocyclic carbonyl group, a hydroxy group, a halogen atom, an aromatic group, an aliphatic oxy group, a carbamoyl group, an aliphatic oxycarbonyl group, an aliphatic thio group, an amino group, an aliphatic amino group, an acylamino group, a carbamoylamino group, or the like. Examples of the acyl group include acyl groups having 2 to 8 and preferably 2 to 4 carbon atoms in total, such as an acetyl group, a propanoyl group, a benzoyl group, and a 3-pyridinecarbonyl group.

The acylamino group may have an aliphatic group, an aromatic group, a heterocyclic group, or the like, and may have, for example, an acetylamino group, a benzoylamino group, a 2-pyridinecarbonylamino group, a propanoylamino group, or the like. Examples of the acylamino group include acylamino groups having 2 to 12 and preferably 2 to 8 carbon atoms in total and alkylcarbonylamino groups having 2 to 8 carbon atoms in total, such as an acetylamino group, a benzoylamino group, a 2-pyridinecarbonylamino group, and a propanoylamino group.

The aliphatic oxycarbonyl group may be saturated or unsaturated, and may have a hydroxy group, an aliphatic oxy group, a carbamoyl group, an aliphatic oxycarbonyl group, an aliphatic thio group, an amino group, an aliphatic amino group, an acylamino group, a carbamoylamino group, or the like. Examples of the aliphatic oxycarbonyl group include alkoxycarbonyl groups having 2 to 8 and preferably 2 to 4 carbon atoms in total, such as a methoxycarbonyl group, an ethoxycarbonyl group, and a (t)-butoxycarbonyl group.

The carbamoyl group may have an aliphatic group, an aromatic group, a heterocyclic group, or the like. Examples of the carbamoyl group include an unsubstituted carbamoyl group and alkylcarbamoyl groups having 2 to 9 carbon atoms in total, and preferably an unsubstituted carbamoyl group and alkylcarbamoyl groups having 2 to 5 carbon atoms in total, such as a N-methylcarbamoyl group, a N,N-dimethylcarbamoyl group, and a N-phenylcarbamoyl group.

The aliphatic sulfonyl group may be saturated or unsaturated, and may have a hydroxy group, an aromatic group, an aliphatic oxy group, a carbamoyl group, an aliphatic oxycarbonyl group, an aliphatic thio group, an amino group, an aliphatic amino group, an acylamino group, a carbamoylamino group, or the like. Examples of the aliphatic sulfonyl group include alkylsulfonyl groups having 1 to 6 carbon atoms in total and preferably 1 to 4 carbon atoms in total, such as a methanesulfonyl group.

The aromatic sulfonyl group may have a hydroxy group, an aliphatic group, an aliphatic oxy group, a carbamoyl group, an aliphatic oxycarbonyl group, an aliphatic thio group, an amino group, an aliphatic amino group, an acylamino group, a carbamoylamino group, or the like. Examples of the aromatic sulfonyl group include arylsulfonyl groups having 6 to 10 carbon atoms in total, such as a benzenesulfonyl group.

The amino group may have an aliphatic group, an aromatic group, a heterocyclic group, or the like.

The acylamino group may have, for example, an acetylamino group, a benzoylamino group, a 2-pyridinecarbonylamino group, a propanoylamino group, or the like. Examples of the acylamino group include acylamino groups having 2 to 12 carbon atoms in total and preferably 2 to 8 carbon atoms in total, and more preferably alkylcarbonylamino groups having 2 to 8 carbon atoms in total, such as an acetylamino group, a benzoylamino group, a 2-pyridinecarbonylamino group, and a propanoylamino group.

The aliphatic sulfonamide group, the aromatic sulfonamide group, and the heterocyclic sulfonamide group may be, for example, a methanesulfonamide group, a benzenesulfonamide group, and a 2-pyridinesulfonamide group, respectively.

The sulfamoyl group may have an aliphatic group, an aromatic group, a heterocyclic group, or the like. Examples of the sulfamoyl group include a sulfamoyl group, alkylsulfamoyl groups having 1 to 9 carbon atoms in total, dialkylsulfamoyl groups having 2 to 10 carbon atoms in total, arylsulfamoyl groups having 7 to 13 carbon atoms in total, and heterocyclic sulfamoyl groups having 2 to 12 carbon atoms in total, more preferably a sulfamoyl group, alkylsulfamoyl groups having 1 to 7 carbon atoms in total, dialkylsulfamoyl groups having 3 to 6 carbon atoms in total, arylsulfamoyl groups having 6 to 11 carbon atoms in total, and heterocyclic sulfamoyl groups having 2 to 10 carbon atoms in total, such as a sulfamoyl group, a methylsulfamoyl group, a N,N-dimethylsulfamoyl group, a phenylsulfamoyl group, and a 4-pyridinesulfamoyl group.

The aliphatic oxy group may be saturated or unsaturated, and may have a methoxy group, an ethoxy group, an i-propyloxy group, a cyclohexyloxy group, a methoxyethoxy group, or the like. Examples of the aliphatic oxy group include alkoxy groups having 1 to 8 and preferably 1 to 6 carbon atoms in total, such as a methoxy group, an ethoxy group, an i-propyloxy group, a cyclohexyloxy group, and a methoxyethoxy group.

The aromatic amino group and the heterocyclic amino group may have an aliphatic group, an aliphatic oxy group, a halogen atom, a carbamoyl group, a heterocyclic group having a ring condensed with the aryl group, or an aliphatic oxycarbonyl group, and preferably an aliphatic group having 1 to 4 carbon atoms in total, an aliphatic oxy group having 1 to 4 carbon atoms in total, a halogen atom, a carbamoyl group having 1 to 4 carbon atoms in total, a nitro group, or an aliphatic oxycarbonyl group having 2 to 4 carbon atoms in total.

The aliphatic thio group may be saturated or unsaturated, and examples include alkylthio groups having 1 to 8 carbon atoms in total and more preferably 1 to 6 carbon atoms in total, such as a methylthio group, an ethylthio group, a carbamoylmethylthio group, and a t-butylthio group.

The carbamoylamino group may have an aliphatic group, an aryl group, a heterocyclic group, or the like. Examples of the carbamoylamino group include a carbamoylamino group, alkylcarbamoylamino groups having 2 to 9 carbon atoms in total, dialkylcarbamoylamino groups having 3 to 10 carbon atoms in total, arylcarbamoylamino groups having 7 to 13 carbon atoms in total, and heterocyclic carbamoylamino groups having 3 to 12 carbon atoms in total, and preferably a carbamoylamino group, alkylcarbamoylamino groups having 2 to 7 carbon atoms in total, dialkylcarbamoylamino groups having 3 to 6 carbon atoms in total, arylcarbamoylamino groups having 7 to 11 carbon atoms in total, and heterocyclic carbamoylamino groups having 3 to 10 carbon atoms in total, such as a carbamoylamino group, a methylcarbamoylamino group, a N,N-dimethylcarbamoylamino group, a phenylcarbamoylamino group, and a 4-pyridinecarbamoylamino group.

A range indicated by endpoints as used herein includes all numerical values within the range (for example, the range of 1 to 10 includes 1.4, 1.9, 2.33, 5.75, 9.98, and the like).

The phrase "at least one" as used herein includes all numerical values equal to or greater than 1 (for example, at least 2, at least 4, at least 6, at least 8, at least 10, at least 25, at least 50, at least 100, and the like).

Hereinafter, specific embodiments of the present disclosure will now be described in detail, but the present disclosure is not limited to the following embodiments.

The compound of the present disclosure is a compound represented by general formula:

R¹-R²-X

wherein R¹ is -CH₃, -CH₂F, -CHF₂, -CH₂I, -CHFI, or an anionic group; R² is an alkylene group consisting of a unit represented by -CFH-, or an alkylene group consisting of a unit represented by -CFH- and a unit represented by -CH₂-, provided that these alkylene groups optionally contain an epoxy group, -CH(OH)-, -CHI-, or a divalent cycloalkylene group; X is -OH, -CH(R²¹)OH (wherein R²¹ is H, a non-fluorinated alkyl group, or a fluorinated alkyl group), -I, -CFHI, -CH₂I, an anionic group, or -COOR²² (wherein R²² is a non-fluorinated alkyl group having 1 to 8 carbon atoms); and the total number of carbon atoms of R¹, R², and X is 2 to 50.

R¹ is -CH₃, -CH₂F, -CHF₂, -CH₂I, -CHFI, or an anionic group; preferably -CH₂F, -CHF₂, or -CHFI, and even more preferably -CHF₂. The compound of the present disclosure has a feature of having no CF₃- (trifluoromethyl group) at a molecular end.

R² is an alkylene group consisting of a unit represented by -CFH-, or an alkylene group consisting of a unit represented by -CFH- and a unit represented by -CH₂-. The compound of the present disclosure has a feature of certainly containing a unit represented by -CFH- in the molecular chain and containing no unit represented by -CF₂- in the molecular chain.

The number of carbon atoms of R² is preferably 1 or more, more preferably 2 or more, and even more preferably 3 or more, and preferably 49 or less, more preferably 15 or less, and even more preferably 11 or less.

The alkylene group in R² optionally contains epoxy group, -CH(OH)-, -CHI- or a divalent cycloalkylene group. That is, a part of the alkylene group in R² may be replaced by epoxy group (oxirane group) or a cycloalkylene group, and any one of H and F bonded to a carbon atom constituting the alkylene group in R² may be replaced by OH or I.

When R² is an alkylene group consisting of a unit represented by -CFH-, examples of R² include -(CFH)ₙ₁- wherein n1 is an integer of 1 or more, preferably an alkylene group represented by -(CFH)ₙ₁- wherein n1 is an integer of 1 to 49, and more preferably an alkylene group represented by -CHF-(CHF-CHF)ₙ- wherein n is an integer of 0 to 24 or an alkylene group represented by -(CHF-CHF)ₙ- wherein n is an integer of 1 to 24. n1 is preferably an integer of 1 to 15, even more preferably an integer of 3 to 11. n is preferably an integer of 1 to 7, and even more preferably an integer of 1 to 5.

When R² is an alkylene group consisting of a unit represented by -CFH- and a unit represented by -CH₂-, examples of R² include an alkylene group represented by -CHF-(CHF-CHF)ₙ-(CH₂)ₘ- wherein n is an integer of 0 or more and m is an integer of 1 or more; an alkylene group represented by -(CHF)ₚ-(CH₂)_{q}- wherein p and q are independently an integer of 1 or more, the total number of p and q is 2 to 49, the occurrence order of the unit represented by -CFH- and the unit represented by -CH₂- is arbitrary in the formula, and any one of H and F bonded to a carbon atom is optionally replaced by OH; and an alkylene group represented by -CHF-(CHF-CHF)ₙ-CH₂-CHI-(CH₂)_{q}- wherein n is an integer of 0 or more, and q is an integer of 1 or more.

When R² is an alkylene group consisting of a unit represented by -CFH- and a unit represented by -CH₂-, R² is preferably an alkylene group represented by -CHF-(CHF-CHF)ₙ-(CH₂)ₘ- wherein n is an integer of 1 to 24 and m is an integer of 1 or more, or an alkylene group represented by -CHF-(CHF-CHF)ₙ-CH₂-CHI-(CH₂)_{q}- wherein n is an integer of 0 or more and q is an integer of 1 or more, and more preferably an alkylene group represented by -CHF-(CHF-CHF)ₙ-CH₂- wherein n is an integer of 1 to 24, -CHF-(CHF-CHF)ₙ-CH₂CH₂- wherein n is an integer of 1 to 23, or an alkylene group represented by -CHF-(CHF-CHF)ₙ-CH₂-CHI-(CH₂)_{q}- wherein n is an integer of 0 or more and q is an integer of 1 or more. n is preferably an integer of 0 to 6, more preferably an integer of 0 to 4, and even more preferably an integer of 1 to 3. m is preferably 1 or 2. q is preferably an integer of 1 to 24, more preferably an integer of 1 to 18, and even more preferably an integer of 1 to 12.

X is -OH, -CH(R²¹)OH wherein R²¹ is H, a non-fluorinated alkyl group, or a fluorinated alkyl group, -I, -CFHI, -CH₂I, an anionic group, or -COOR²² wherein R²² is a non-fluorinated alkyl group having 1 to 8 carbon atoms. The anionic group preferably contains -COOM, -SO₃M or -OSO₃M. Examples of the anionic group include -COOM, -SO₃M, -CH(R²¹)-O-(CH₂)₃-SO₃M, -OSO₃M, and - CH(R²¹)-OSO₃M.

M in the anionic group represents a counter cation of an anion. M is preferably H, a metal atom, NR⁷₄, optionally substituted imidazolium, optionally substituted pyridinium, or optionally substituted phosphonium. R⁷ is preferably H or an organic group.

Examples of the metal atom include alkali metals (Group 1) and alkaline earth metals (Group 2), and Na, K, or Li is preferable.

M is preferably -H, a metal atom, or NR⁷₄, more preferably H, an alkali metal (Group 1), an alkaline earth metal (Group 2), or NR⁷₄, even more preferably H, Na, K, Li, or NH₄, yet more preferably H, Na, K, or NH₄, particularly preferably H, Na, or NH₄, and most preferably H or NH₄.

R²¹ is H, a non-fluorinated alkyl group, or a fluorinated alkyl group, preferably H or a non-fluorinated alkyl group having 1 to 3 carbon atoms, and more preferably H.

R²² is a non-fluorinated alkyl group having 1 to 8 carbon atoms, preferably a non-fluorinated alkyl group having 1 to 4 carbon atoms, and more preferably -CH₃ or -CH₂CH₃.

The total number of carbon atoms of R¹, R², and X is 2 to 50, preferably 2 to 16, more preferably 4 to 14, even more preferably 4 to 12, particularly preferably 4 to 10, and most preferably 4 to 8.

More specific examples of the compound of the present disclosure include the following compounds:
a fluorine-containing alcohol represented by general formula: CH₂F-CHF-(CHF-CHF)ₙ-CH(R²¹)OH
   wherein n is an integer of 0 or more and R²¹ is as described above;
a fluorine-containing carboxylic acid represented by general formula: CH₂F-CHF-(CHF-CHF)ₙ-COOM
   wherein n is an integer of 0 or more and M is a cation;
a fluorine-containing sulfate represented by general formula: CH₂F-CHF-(CHF-CHF)ₙ-CH(R²¹)-OSO₃M
   wherein n is an integer of 0 or more, R²¹ is as described above, and M is a cation;
the first, second, fourth, or fifth fluorine-containing alkyl iodide represented by general formula: R¹-CHF-(CHF-CHF)ₙ-I
   wherein R¹ is as described above and n is an integer of 0 or more (n is preferably an integer of 1 or more);
the third fluorine-containing alkyl iodide represented by general formula: R¹-CHF-(CHF-CHF)ₙ-CH₂CH₂-I
   wherein R¹ is as described above and n is an integer of 1 or more;
a fluorine-containing alcohol represented by general formula: R¹-CHF-(CHF-CHF)ₙ-CH₂CH₂-OH
   wherein R¹ is as described above and n is an integer of 0 or more;
a fluorine-containing carboxylic acid represented by general formula: R¹-CHF-(CHF-CHF)ₙ-CH₂-COOM
   wherein R¹ is as described above, n is an integer of 0 or more, and M is a cation;
a fluorine-containing carboxylic acid derivative represented by general formula: R¹-CHF-(CHF-CHF)ₙ-(CH2)ₘ-COOR²³ wherein R¹ is as described above, n is an integer of 0 or more, m is an integer of 0 to **3,** and R²³ is H or an alkyl group having 1 to 8 carbon atoms;
an oligomer represented by general formula: CH₂F-R²-OSO₃M wherein R² is an alkylene group represented by -(CFH)ₙ₁-(wherein n1 is an integer of 3 to 49), and M is a cation;
an oligomer represented by general formula: R²(-OSO₃M)₂ wherein R² is an alkylene group represented by -(CFH)ₙ₁-(wherein n1 is an integer of 1 to 49), and M is a cation;
a fluoride of an unsaturated fatty acid represented by general formula: CH₃-R²-COOM
   wherein R² is an alkylene group consisting of a unit represented by -CFH- or an alkylene group consisting of a unit represented by -CFH- and a unit represented by -CH₂-, provided that these alkylene groups optionally contain an epoxy group, -CH(OH)-, or a divalent cycloalkylene group, and M is a cation;
an iodine-containing compound represented by general formula: R¹-CHF-(CHF-CHF)ₙ-CH₂-CHI-(CH₂)_{q}-X
   wherein R¹ is -CHF₂ or -CHFI, X is -OH, -COOM (wherein M is a cation), -SO₃M (wherein M is a cation), -OSO₃M (wherein M is a cation), or -COOR²² (wherein R²² is a non-fluorinated alkyl group having 1 to 8 carbon atoms), n is an integer of 0 or more, and q is an integer of 1 or more; and
a compound represented by general formula: R¹-CHF-(CHF-CHF)ₙ-CH₂-CH₂- (CH₂)_{q}-X
   wherein R¹ is -CHF₂ or -CHFI, X is -OH, -COOM (wherein M is a cation), -SO₃M (wherein M is a cation), -OSO₃M (wherein M is a cation), or -COOR²² (wherein R²² is a non-fluorinated alkyl group having 1 to 8 carbon atoms), n is an integer of 0 or more, and q is an integer of 1 or more.

As described above, the compound of the present disclosure includes the fluorine-containing alcohol, the fluorine-containing carboxylic acid, the fluorine-containing sulfate, the fluorine-containing alkyl iodide, the oligomer, the fluoride of an unsaturated fatty acid, the iodine-containing compound, and the like. Next, production methods of these compounds will be described.

### <First production method>

In the first production method, the fluorine-containing alcohol represented by the general formula: CH₂F-CHF-(CHF-CHF)ₙ-CH(R²¹)OH wherein n is an integer of 0 or more and R²¹ is as described above is produced by adding CHF=CHF to an alkanol represented by general formula: R²¹-CH₂-OH wherein R²¹ is H, a non-fluorinated alkyl group, or a fluorinated alkyl group.

R²¹ in the alkanol and the fluorine-containing alcohol is H, a non-fluorinated alkyl group, or a fluorinated alkyl group, preferably H or a non-fluorinated alkyl group having 1 to 3 carbon atoms, and more preferably H.

n in the fluorine-containing alcohol represents the degree of polymerization of CHF=CHF, and is an integer of 0 or more. n is, for example, an integer of 0 to 23, preferably an integer of 1 to 7, more preferably an integer of 1 to 5, and even more preferably an integer of 1 to 3.

The reaction of the alkanol with CHF=CHF can be performed in the presence of a radical initiator. When the reaction is performed in the presence of a radical initiator, the radical initiator is decomposed to generate radicals, a generated radical draws out a hydrogen atom on the carbon atom to which the hydroxyl group of the alkanol is bonded, to generate an alkanol radical, and a reaction of adding CHF=CHF to the alkanol radical (a so-called telomerization reaction) proceeds.

The radical initiator is preferably an organic peroxide, and preferable examples thereof include dialkyl peroxycarbonates such as diisopropyl peroxydicarbonate and di sec-butyl peroxydicarbonate; peroxyesters such as 2-ethylhexanoyl (tert-butyl) peroxide, t-butyl peroxyisobutyrate, and t-butyl peroxypivalate; and dialkyl peroxides such as di t-butyl peroxide.

The amount of CHF=CHF used is preferably 0.01 to 100 mol per 1 mol of the alkanol.

The amount of the radical initiator used is preferably 0.01 to 2 mol per 1 mol of the alkanol.

The reaction temperature of the alkanol with CHF=CHF can be suitably selected, and is preferably -78 to 200°C. The reaction temperature of the alkanol with CHF=CHF is preferably the decomposition temperature of the radical polymerization initiator or more, and preferably less than the decomposition temperatures of the substrate and the product.

The reaction pressure of the alkanol with CHF=CHF can be suitably selected, and is preferably 0 to 5.0 MPaG. The reaction time of the alkanol with CHF=CHF can be suitably selected, and is preferably 0.1 to 96 hours.

### <Second production method>

In the second production method, the fluorine-containing carboxylic acid represented by the general formula: CH₂F-CHF-(CHF-CHF)ₙ-COOM wherein n is an integer of 0 or more and M is a cation is produced by producing the fluorine-containing alcohol represented by the general formula: CH₂F-CHF-(CHF-CHF)ₙ-CH(R²¹)OH wherein n is an integer of 0 or more and R²¹ is as described above by the first production method, and then oxidizing the fluorine-containing alcohol when R²¹ is H.

n in the fluorine-containing carboxylic acid has the same value as n in the fluorine-containing alcohol, and is an integer of 0 or more. The preferable range of n in the fluorine-containing carboxylic acid is the same as the preferable range of n in the fluorine-containing alcohol.

M is a counter cation of -COO⁻. Examples thereof include the same cations as M contained in the anionic group as X described above, and the same applies to the preferable cation.

The oxidation of the fluorine-containing alcohol can be performed in the presence of an oxidizing agent. Examples of the oxidizing agent include potassium permanganate. The amount of the oxidizing agent used is preferably 0.01 to 100 mol per 1 mol of the fluorine-containing alcohol.

The oxidation of the fluorine-containing alcohol can be performed in a solvent. Examples of the solvent include water.

The oxidation temperature of the fluorine-containing alcohol can be suitably selected, and is preferably -78 to 200°C. The oxidation pressure of the fluorine-containing alcohol can be suitably selected, and is preferably 0 to 5.0 MPaG. The oxidation time of the fluorine-containing alcohol can be suitably selected, and is preferably 0.1 to 96 hours.

### <Third production method>

In the third production method, the fluorine-containing sulfate represented by the general formula: CH₂F-CHF-(CHF-CHF)ₙ-CH(R²¹)-OSO₃M wherein n is an integer of 0 or more, R²¹ is as described above, and M is a cation is produced by producing the fluorine-containing alcohol represented by the general formula: CH₂F-CHF-(CHF-CHF)ₙ-CH(R²¹)OH wherein n is an integer of 0 or more and R²¹ is as described above by the first production method, and then reacting the fluorine-containing alcohol and chlorosulfuric acid.

R²¹ in the fluorine-containing sulfate is the same as R²¹ in the fluorine-containing alcohol, and is a single bond, a non-fluorinated alkylene group, or a fluorinated alkylene group. The preferable group of R²¹ in the fluorine-containing sulfate is the same as the preferable group of R²¹ in the fluorine-containing alcohol.

n in the fluorine-containing sulfate has the same value as n in the fluorine-containing alcohol, and is an integer of 0 or more. The preferable range of n in the fluorine-containing sulfate is the same as the preferable range of n in the fluorine-containing alcohol.

M is a counter cation of -OSO₃⁻. Examples thereof include the same cations as M contained in the anionic group as X described above, and the same applies to the preferable cation.

The amount of chlorosulfuric acid used is preferably 1 to 2 mol per 1 mol of the fluorine-containing alcohol.

The reaction of the fluorine-containing alcohol with chlorosulfuric acid can be performed in the presence of a base. Examples of the base include alkali metal hydroxides, alkaline earth metal hydroxides, and amines, and in particular, amines are preferable.

Examples of the amine include tertiary amines such as trimethylamine, triethylamine, tributylamine, **N,N-**dimethylaniline, dimethylbenzylamine, and N,N,N',N'-tetramethyl-1,8-naphthalene diamine; heteroaromatic amines such as pyridine, pyrrole, uracil, collidine, and lutidine; and cyclic amines such as 1,8-diaza-bicyclo[5.4.0]-7-undecene and 1,5- diaza-bicyclo[4.3.0]-5-nonene. In particular, triethylamine or pyridine is preferable.

The amount of the base used is 0.5 to 20 mol per 1 mol of the fluorine-containing alcohol.

The reaction of the fluorine-containing alcohol with chlorosulfuric acid can be performed in a solvent. The solvent is preferably a polar solvent, more preferably an aprotic polar solvent, and even more preferably an ether.

Examples of the ether include ethyl methyl ether, diethyl ether, monoglyme (ethylene glycol dimethyl ether), diglyme (diethylene glycol dimethyl ether), triglyme (triethylene glycol dimethyl ether), tetrahydrofuran, tetraglyme (tetraethylene glycol dimethyl ether), and crown ether (15-crown-5,18-crown-6), and in particular, diethyl ether is preferable.

The reaction temperature of the fluorine-containing alcohol with chlorosulfuric acid can be suitably selected, and is preferably 0 to 40°C. The reaction pressure of the fluorine-containing alcohol with chlorosulfuric acid can be suitably selected, and is preferably 0.1 to 5 MPaG. The reaction time of the fluorine-containing alcohol with chlorosulfuric acid can be suitably selected, and is preferably 0.1 to 96 hours.

### <Fourth production method>

In the fourth production method, the second fluorine-containing alkyl iodide represented by the general formula: R¹-CHF-(CHF-CHF)ₙ-I wherein R¹ is as described above and n is an integer of 1 or more is produced by reacting CHF=CHF with an iodide compound represented by general formula: X¹I wherein X¹ is H or F to produce the first fluorine-containing alkyl iodide represented by general formula: R¹-CHF-I wherein R¹ is - CH₂F or -CHF₂, and adding CHF=CHF to the first fluorine-containing alkyl iodide.

The amount of the iodide compound used is preferably 0.5 to 2 mol per 1 mol of CHF=CHF.

The reaction of CHF=CHF with the iodide compound can also be performed in a solvent.

The reaction temperature of CHF=CHF with the iodide compound can be suitably selected, and is preferably -78 to 200°C. The reaction pressure of CHF=CHF with the iodide compound can be suitably selected, and is preferably 0 to 5.0 MPaG. The reaction time of CHF=CHF with the iodide compound can be suitably selected, and is preferably 0.1 to 96 hours.

The first fluorine-containing alkyl iodide represented by the general formula: R¹-CHF-I wherein R¹ is -CH₂F or -CHF₂ is produced by the reaction of CHF=CHF with the iodide compound. In the fourth production method, CHF=CHF is then added to the first fluorine-containing alkyl iodide.

The reaction of the first fluorine-containing alkyl iodide with CHF=CHF is a telomerization reaction in which the first fluorine-containing alkyl iodide is a telogen and CHF=CHF is a taxogen, and this reaction produces the second fluorine-containing alkyl iodide.

n in the second fluorine-containing alkyl iodide represents the degree of polymerization of CHF=CHF, and is an integer of 1 or more. n is preferably an integer of 1 to 23, more preferably an integer of 1 to 7, even more preferably an integer of 1 to 5, and particularly preferably an integer of 1 to 3.

The reaction of the first fluorine-containing alkyl iodide with CHF=CHF can be performed in the presence of a radical initiator. Examples of the radical initiator include organic peroxides and azo compounds.

Examples of the organic peroxide include dialkyl peroxycarbonates such as diisopropyl peroxydicarbonate and di sec-butyl peroxydicarbonate; peroxyesters such as 2-ethylhexanoyl (tert-butyl) peroxide, t-butyl peroxyisobutyrate, and t-butyl peroxypivalate; and dialkyl peroxides such as di t-butyl peroxide.

Examples of the azo compound include azobisisobutyronitrile.

The amount of CHF=CHF used is preferably 0.01 to 100 mol per 1 mol of the fluorine-containing alkyl iodide.

The amount of the radical initiator used is preferably 0.01 to 2 mol per 1 mol of the fluorine-containing alkyl iodide.

The reaction temperature of the first fluorine-containing alkyl iodide with CHF=CHF can be suitably selected, and is preferably -78 to 200°C. The reaction temperature of the first fluorine-containing alkyl iodide with CHF=CHF is preferably the decomposition temperature of the radical polymerization initiator or more, and is preferably less than the decomposition temperatures of the substrate and the product.

The reaction pressure of the first fluorine-containing alkyl iodide with CHF=CHF can be suitably selected, and is preferably 0 to 5.0 MPaG. The reaction time of the first fluorine-containing alkyl iodide with CHF=CHF can be suitably selected, and is preferably 0.1 to 96 hours.

### <Fifth production method>

In the fifth production method, the third fluorine-containing alkyl iodide represented by the general formula: R¹-CHF-(CHF-CHF)ₙ-CH₂CH₂-I wherein R¹ is as described above and n is an integer of 0 or more is produced by producing the first or second fluorine-containing alkyl iodide by the fourth production method, and then adding ethylene to the first or second fluorine-containing alkyl iodide.

R¹ in the third fluorine-containing alkyl iodide is the same as R¹ in the first or second fluorine-containing alkyl iodide, and is -CH₂F or -CHF₂.

n in the third fluorine-containing alkyl iodide is an integer of 0 or more. The preferable range of n in the third fluorine-containing alkyl iodide is an integer of 0 to 23, preferably an integer of 0 to 7, more preferably an integer of 0 to 5, and even more preferably an integer of 0 to 3.

The reaction of the first or second fluorine-containing alkyl iodide with ethylene can be performed in the presence of a metal catalyst. Examples of the metal catalyst include copper.

The reaction of the first or second fluorine-containing alkyl iodide with ethylene can be performed in the presence of a compound generating radicals. Examples of such a compound include organic peroxides and azo compounds.

Examples of the organic peroxide include dialkyl peroxycarbonates such as diisopropyl peroxydicarbonate and di sec-butyl peroxydicarbonate; peroxyesters such as 2-ethylhexanoyl (tert-butyl) peroxide, t-butyl peroxyisobutyrate, and t-butyl peroxypivalate; and dialkyl peroxides such as di t-butyl peroxide.

Examples of the azo compound include azobisisobutyronitrile.

The amount of ethylene used is preferably 0.01 to 100 mol per 1 mol of the first or second fluorine-containing alkyl iodide.

The amount of the compound generating radicals used is preferably 0.001 to 1 mol per 1 mol of the first or second fluorine-containing alkyl iodide.

The reaction temperature of the first or second fluorine-containing alkyl iodide with ethylene can be suitably selected, and is preferably 50 to 200°C. The reaction pressure of the first or second fluorine-containing alkyl iodide with ethylene can be suitably selected, and is preferably 0.1 to 5 MPaG. The reaction time of the first or second fluorine-containing alkyl iodide with ethylene can be suitably selected, and is preferably 0.1 to 96 hours.

### <Sixth production method>

In the sixth production method, the fluorine-containing alcohol represented by the general formula: R¹-CHF-(CHF-CHF)ₙ-CH₂CH₂-OH wherein R¹ is as described above and n is an integer of 0 or more is produced by producing the third fluorine-containing alkyl iodide represented by the general formula: R¹-CHF-(CHF-CHF)ₙ-CH₂CH₂-I wherein R¹ is as described above and n is an integer of 0 or more by the fifth production method, then reacting the third fluorine-containing alkyl iodide with a fuming sulfuric acid, and then subjecting the resulting product to hydrolysis.

R¹ in the fluorine-containing alcohol is the same as R¹ in the third fluorine-containing alkyl iodide, and is -CH₂F or -CHF₂ .

n in the fluorine-containing alcohol has the same value as n in the third fluorine-containing alkyl iodide, and is an integer of 0 or more. The preferable range of n in the fluorine-containing alcohol is the same as the preferable range of n in the third fluorine-containing alkyl iodide.

The content of sulfur trioxide in the fuming sulfuric acid is not limited, and is preferably 10 to 90% by mass, more preferably 30 to 80% by mass, and even more preferably 50 to 70% by mass.

The amount of the fuming sulfuric acid used is, as an amount equivalent to sulfur trioxide in the fuming sulfuric acid, preferably 1 to 50 mol per 1 mol of the third fluorine-containing alkyl iodide.

The reaction temperature of the third fluorine-containing alkyl iodide with the fuming sulfuric acid can be suitably selected, and is preferably 0 to 90°C. The reaction pressure of the third fluorine-containing alkyl iodide with the fuming sulfuric acid can be suitably selected, and is preferably 0 to 10.0 MPaG. The reaction time of the third fluorine-containing alkyl iodide with the fuming sulfuric acid can be suitably selected, and is preferably 0.1 to 96 hours.

In the sixth production method, the reaction of the third fluorine-containing alkyl iodide with the fuming sulfuric acid produces a fluorine-containing alkylhydrogen sulfate represented by general formula: R¹-CHF-(CHF-CHF)ₙ-CH₂CH₂-OSO₃H wherein R¹ is as described above and n is an integer of 0 or more, and the fluorine-containing alkylhydrogen sulfate is then hydrolyzed to produce the fluorine-containing alcohol represented by the general formula: R¹-CHF-(CHF-CHF)ₙ-CH₂CH₂-OH wherein R¹ is as described above and n is an integer of 0 or more.

The hydrolysis of the fluorine-containing alkylhydrogen sulfate can be performed by using, for example, water or an aqueous sodium sulfite solution. The hydrolysis of the fluorine-containing alkylhydrogen sulfate can be performed by, for example, dropwise adding the aqueous sodium sulfite solution to a solution obtained through the reaction of the third fluorine-containing alkyl iodide with the fuming sulfuric acid (fluorine-containing alkylhydrogen sulfate-containing solution).

The amount of water or the aqueous sodium sulfite solution used is not limited, for example, as long as it is an amount capable of neutralizing the solution obtained through the reaction of the third fluorine-containing alkyl iodide with fuming sulfuric acid and required for hydrolyzing the fluorine-containing alkylhydrogen sulfate.

The hydrolysis temperature can be suitably selected, and is preferably 15 to 100°C. The hydrolysis time can be suitably selected, and is preferably 0.1 to 96 hours.

### <Seventh production method>

In the seventh production method, the fluorine-containing carboxylic acid derivative represented by general formula: R¹-CHF-(CHF-CHF)ₙ-(CH2)ₘ-COOR²³ wherein R¹ is as described above, n is an integer of 0 or more, m is an integer of 0 to 3, and R²³ is H or an alkyl group having 1 to 8 carbon atoms is produced by reacting the first, second, or third fluorine-containing alkyl iodide with carbon dioxide or a dialkyl carbonate.

The fluorine-containing carboxylic acid derivative can be produced by reacting the first, the second, or third fluorine-containing alkyl iodide with carbon dioxide or a dialkyl carbonate in the presence of a base.

The reaction can be performed in the presence of a base. Examples of the base include alkali metal hydroxides, alkaline earth metal hydroxides, and amines. Examples of the amine include aliphatic amines such as tributylamine.

The amount of the base used is 0.5 to 20 mol per 1 mol of the fluorine-containing alkyl iodide.

The temperature when the reaction of the fluorine-containing alkyl iodide with carbon dioxide is performed can be suitably selected, and is preferably -78 to 200°C.

When the fluorine-containing alkyl iodide is reacted with a dialkyl carbonate, for example, dimethyl carbonate can be used as the dialkyl carbonate. The reaction of the fluorine-containing alkyl iodide with dimethyl carbonate progresses a methoxycarbonylation reaction, so that a corresponding carboxylate is produced.

When a carboxylate in which R²³ is an alkyl group is produced as the carboxylic acid derivative, the carboxylate can be converted to a carboxylic acid or a carboxylic salt by the hydrolysis.

### <Eighth production method>

In the eighth production method, the oligomer represented by the general formula: CH₂F-R²-OSO₃M wherein R² is an alkylene group represented by -(CFH)ₙ₁- (wherein n1 is an integer of 1 to 49), and M is a cation, or the oligomer represented by the general formula: R²(-OSO₃M)₂ wherein R² is an alkylene group represented by -(CFH)ₙ₁- (wherein n1 is an integer of 1 to 49), and M is a cation is produced by polymerizing CHF=CHF in the presence of a persulfate.

R² is an alkylene group represented by -(CFH)ₙ₁-, n1 is an integer of 1 to 49, and is preferably an integer of 3 to 49. That is, the oligomer obtained by the eighth production method is a low-molecular weight compound containing a repeating unit derived from CHF=CHF, and the number of repeating units derived from CHF=CHF is 1 to 25. For example, the oligomer obtained by the eighth production method may be an oligomer having a molecular weight distribution, and in this case, n1 represents the average number of repeating units of the molecule contained in the oligomer.

M is a counter cation of -OSO₃⁻. Examples thereof include the same cations as M contained in the anionic group as X described above, and the same applies to the preferable cation.

The polymerization of CHF=CHF can be performed in the presence of a persulfate. By using a persulfate, the oligomerization of CHF=CHF is initiated, and an oligomer in which the main chain is formed of a repeating unit derived from CHF=CHF and -OSO₃M is introduced into the terminal is produced.

Examples of the persulfate include ammonium salts of persulphuric acid, alkali salts of persulphuric acid, and alkaline earth metal salts of persulphuric acid. The amount of the persulfate used is preferably 0.01 to 1,000% by mass based on the amount of the oligomer produced.

The persulfate may be used in combination with a reducing agent. Examples of the reducing agent include sulfites such as sodium sulfite and sodium bisulfite, metabisulfites such as sodium bisulfite and potassium bisulfite, pyrosulfates, and thiosulfates.

The polymerization of CHF=CHF can be performed in an aqueous medium. The polymerization of CHF=CHF is preferably performed in the absence of a surfactant.

The polymerization temperature of CHF=CHF can be suitably selected, and is preferably -78 to 200°C. The polymerization temperature of CHF=CHF is preferably the decomposition temperature of the persulfate or more, and preferably less than the decomposition temperatures of the substrate and the product.

The polymerization pressure of CHF=CHF can be suitably selected, and is preferably 0 to 5.0 MPaG. The polymerization time of CHF=CHF can be suitably selected, and is preferably 0.1 to 96 hours.

### <Ninth production method>

In the ninth production method, a fluoride of an unsaturated fatty acid represented by the general formula: CH₃-R²-COOM wherein R² is an alkylene group consisting of a unit represented by -CFH- or an alkylene group consisting of a unit represented by -CFH- and a unit represented by -CH₂-, provided that these alkylene groups optionally contain an epoxy group, -CH(OH)-, or a divalent cycloalkylene group, and M is a cation is obtained by reacting an unsaturated fatty acid with HF or F₂.

For example, the unsaturated fatty acid may be any one of a monounsaturated fatty acid and a polyunsaturated fatty acid. The number of carbon atoms in the unsaturated fatty acid is preferably 4 to 50, more preferably 4 to 16, and even more preferably 4 to 10.

R² in the fluoride of the unsaturated fatty acid is preferably -(CHF)ₚ-(CH₂)_{q}- wherein p is an integer of 1 or more, q is an integer of 0 or more, the total number of p and q is 1 to 49, the occurrence order of the unit represented by -CFH- and the unit represented by -CH₂- is arbitrary in the formula, and any one of H and F bonded to a carbon atom is optionally replaced by OH.

M is a counter cation of -COO⁻. Examples thereof include the same cations as M contained in the anionic group as X described above, and the same applies to the preferable cation.

Examples of the unsaturated fatty acid include the following compounds:
monounsaturated fatty acids such as crotonic acid, myristoleic acid, palmitoleic acid, sapienic acid, oleic acid, elaidic acid, vaccenic acid, gadoleic acid, eicosenoic acid, erucic acid, and nervonic acid;
diunsaturated fatty acids such as linoleic acid, eicosadienoic acid, and docosadienoic acid;
triunsaturated fatty acids such as linolenic acid, pinolenic acid, eleostearic acid, mead acid, dihomo-γ-linolenic acid, and eicosatrienoic acid;
tetraunsaturated fatty acids such as stearidonic acid, arachidonic acid, eicosatetraenoic acid, and adrenic acid;
pentaunsaturated fatty acids such as bosseopentaenoic acid, eicosapentaenoic acid, osbond acid, clupanodonic acid, and tetracosapentaenoic acid;
hexaunsaturated fatty acids such as docosahexaenoic acid and nisinic acid;
epoxides of unsaturated fatty acids such as epoxidized oleic acid and epoxidized linoleic acid; and
eicosanoids such as prostaglandin, leukotriene, and thromboxane.

The reaction of the unsaturated fatty acid with HF or F₂ can be performed by bringing the unsaturated fatty acid into contact with hydrogen fluoride gas or fluorine gas. The reaction may be performed by bringing the unsaturated fatty acid into contact with hydrofluoric acid (an aqueous solution of hydrogen fluoride). By using a catalyst, the reaction can be efficiently performed.

The reaction of the unsaturated fatty acid with HF or F₂ may fluorinate all the unsaturated bonds in the unsaturated fatty acid, or may fluorinate a part of the unsaturated bonds in the unsaturated fatty acid. The fluoride of the unsaturated fatty acid obtained by the tenth production method includes fluorides of the unsaturated fatty acid in which all the unsaturated bonds in the unsaturated fatty acid are fluorinated, and fluorides of the unsaturated fatty acid in which a part of the unsaturated bonds in the unsaturated fatty acid is fluorinated.

The amount of HF or F₂ used is 0.01 to 100 mol per 1 mol of the unsaturated bond in the unsaturated fatty acid.

The reaction temperature of the unsaturated fatty acid with HF or F₂ can be suitably selected, and is preferably -78 to 200°C. The reaction pressure of the unsaturated fatty acid with HF or F₂ can be suitably selected, and is preferably 0 to 5.0 MPaG. The reaction time of the unsaturated fatty acid with HF or F₂ can be suitably selected, and is preferably 0.1 to 96 hours.

### <Tenth production method>

In the tenth production method, the fourth fluorine-containing alkyl iodide represented by general formula: R¹-CHF-I wherein R¹ is -CHF₂ or -CHFI is produced by reacting CHF=CHF with I₂ and IF₅.

The amount of I₂ and IF₅ used is preferably 0.5 to 2 mol per 1 mol of CHF=CHF.

The reaction of CHF=CHF with I₂ and IF₅ can also be performed in a solvent.

The reaction temperature of CHF=CHF with I₂ and IF₅ can be suitably selected, and is preferably -78 to 200°C. The reaction pressure of CHF=CHF with I₂ and IF₅ can be suitably selected, and is preferably 0 to 5.0 MPaG. The reaction time of CHF=CHF with I₂ and IF₅ can be suitably selected, and is preferably 0.1 to 96 hours.

In the tenth production method, the fifth fluorine-containing alkyl iodide represented by the general formula: R¹-CHF-(CHF-CHF)ₙ-I wherein R¹ is as described above, and n is an integer of 1 or more can be produced by further adding CHF=CHF to the fourth fluorine-containing alkyl iodide.

The reaction of the fourth fluorine-containing alkyl iodide with CHF=CHF is a telomerization reaction in which the fourth fluorine-containing alkyl iodide is a telogen and CHF=CHF is a taxogen, and this reaction produces the fifth fluorine-containing alkyl iodide.

n in the fifth fluorine-containing alkyl iodide represents the degree of polymerization of CHF=CHF, and is an integer of 1 or more. n is preferably an integer of 1 to 23, more preferably an integer of 1 to 7, even more preferably an integer of 1 to 5, and particularly preferably an integer of 1 to 3.

The reaction of the fourth fluorine-containing alkyl iodide with CHF=CHF can be performed in the presence of a radical initiator. Examples of the radical initiator include organic peroxides and azo compounds.

Examples of the organic peroxide include dialkyl peroxycarbonates such as diisopropyl peroxydicarbonate and di sec-butyl peroxydicarbonate; peroxyesters such as 2-ethylhexanoyl (tert-butyl) peroxide, t-butyl peroxyisobutyrate, and t-butyl peroxypivalate; and dialkyl peroxides such as di t-butyl peroxide.

Examples of the azo compound include azobisisobutyronitrile.

The amount of CHF=CHF used is preferably 0.01 to 100 mol per 1 mol of the fluorine-containing alkyl iodide.

The amount of the radical initiator used is preferably 0.01 to 2 mol per 1 mol of the fluorine-containing alkyl iodide.

The reaction temperature of the fourth fluorine-containing alkyl iodide with CHF=CHF can be suitably selected, and is preferably -78 to 200°C. The reaction temperature of the fourth fluorine-containing alkyl iodide with CHF=CHF is preferably the decomposition temperature of the radical polymerization initiator or more, and preferably less than the decomposition temperatures of the substrate and the product.

The reaction pressure of the fourth fluorine-containing alkyl iodide with CHF=CHF can be suitably selected, and is preferably 0 to 5.0 MPaG. The reaction time of the fourth fluorine-containing alkyl iodide with CHF=CHF can be suitably selected, and is preferably 0.1 to 96 hours.

### <Eleventh production method>

In the eleventh production method, the iodine-containing compound represented by the general formula: R¹-CHF-(CHF-CHF)ₙ-CH₂-CHI-(CH₂)_{q}-X wherein R¹, X, and q are as described above, and n is an integer of 0 or more is produced by producing the fourth or fifth fluorine-containing alkyl iodide by the tenth production method, and then reacting the fourth or fifth fluorine-containing alkyl iodide with an unsaturated compound represented by general formula: CH₂=CH-(CH₂)_{q}-X wherein X is - OH, -COOM (wherein M is a cation), -SO₃M (wherein M is a cation), -OSO₃M (wherein M is a cation), or -COOR²² (wherein R²² is a non-fluorinated alkyl group having 1 to 8 carbon atoms), and q is an integer of 1 or more.

R¹ in the iodine-containing compound is the same as R¹ in the fourth or fifth fluorine-containing alkyl iodide, and is -CHF₂ or -CHFI.

X in the iodine-containing compound is the same as X in the unsaturated compound, and is -OH, -COOM wherein M is a cation, -SO₃M wherein M is a cation, -OSO₃M wherein M is a cation, or -COOR²² wherein R²² is a non-fluorinated alkyl group having 1 to 8 carbon atoms.

n in the iodine-containing compound is an integer of 0 or more. The preferable range of n in the iodine-containing compound is an integer of 0 to 23, preferably an integer of 0 to 7, more preferably an integer of 0 to 5, and even more preferably an integer of 0 to 3.

q in the iodine-containing compound is an integer of 1 or more. The preferable range of q in the iodine-containing compound is an integer of 1 to 24, preferably an integer of 1 to 18, and more preferably an integer of 1 to 12.

The reaction of the fourth or fifth fluorine-containing alkyl iodide with the unsaturated compound can be performed in the presence of a compound generating radicals. Examples of such a compound include organic peroxides and azo compounds.

Examples of the organic peroxide include dialkyl peroxycarbonates such as diisopropyl peroxydicarbonate and di sec-butyl peroxydicarbonate; peroxyesters such as 2-ethylhexanoyl (tert-butyl) peroxide, t-butyl peroxyisobutyrate, and t-butyl peroxypivalate; and dialkyl peroxides such as di t-butyl peroxide.

Examples of the azo compound include azobisisobutyronitrile.

The amount of the unsaturated compound used is preferably 0.01 to 100 mol per 1 mol of the fourth or fifth fluorine-containing alkyl iodide.

The amount of the compound generating radicals used is preferably 0.001 to 1 mol per 1 mol of the fourth or fifth fluorine-containing alkyl iodide.

The reaction temperature of the fourth or fifth fluorine-containing alkyl iodide with the unsaturated compound can be suitably selected, and is preferably 50 to 200°C. The reaction pressure of the fourth or fifth fluorine-containing alkyl iodide with the unsaturated compound can be suitably selected, and is preferably 0.1 to 5 MPaG. The reaction time of the fourth or fifth fluorine-containing alkyl iodide with the unsaturated compound can be suitably selected, and is preferably 0.1 to 96 hours.

The obtained iodine-containing compound may be reduced to produce a compound represented by the general formula: R¹-CHF-(CHF-CHF)ₙ-CH₂-CH₂-(CH₂)_{q}-X wherein R¹, X, q, and n are as described above. The reduction can be performed by, for example, using a metal catalyst and hydrogen, or using zinc as a reducing agent.

In each production method described above, the purity of the compound to be obtained may be increased by distilling the solvent off, or performing distillation, purification, or the like, after termination of each process. When the compound to be obtained is a compound having an acidic anionic group such as -COOH, -SO₃H, or -OSO₃H, these groups can be converted to basic anionic groups by bringing these groups into contact with an alkali such as sodium carbonate or ammonia.

The compounds of the present disclosure can reduce the surface tension of water. Therefore, the compounds of the present disclosure can be suitably utilized as surfactants. The surfactant of the present disclosure preferably contains at least one compound selected from the group consisting of compounds described above wherein X in the general formula is -OH, -CH(R²¹)OH, or an anionic group.

That is, the surfactant of the present disclosure contains a compound represented by the general formula:

R¹-R²-X

wherein R¹ is -CH₃, -CH₂F, or -CHF₂, R² is an alkylene group consisting of a unit represented by -CFH-, or an alkylene group consisting of a unit represented by -CFH- and a unit represented by -CH₂-, provided that these alkylene groups optionally contain an epoxy group, -CH(OH)-, or a divalent cycloalkylene group, X is -OH, -CH(R²¹)OH (wherein R²¹ is H, a non-fluorinated alkyl group, or a fluorinated alkyl group), or an anionic group, and the total number of carbon atoms of R¹, R², and X is 2 to 50. The surfactant of the present disclosure may contain one or two or more of the above compounds.

The surfactant of the present disclosure can be suitably used in the polymerization of a fluoromonomer. Therefore, the present disclosure includes a method for producing a fluoropolymer in which the fluoropolymer is obtained by performing polymerization a fluoromonomer in an aqueous medium in the presence of the above surfactant. Since the surfactant of the present disclosure contains a unit represented by - CFH-, the surfactant exhibits good interface activity without having a perfluoroalkyl group or a perfluoroalkylene group.

While embodiments have been described above, it will be understood that various changes in form and detail can be made without departing from the gist and scope of the claims.

The main embodiments of the present disclosure are as follows.
<1> According to the first aspect of the present disclosure, provided is a compound represented by general formula:

   R¹-R²-X

   wherein
   R¹ is -CH₃, -CH₂F, -CHF₂, -CH₂I, -CHFI, or an anionic group;
   R² is an alkylene group consisting of unit represented by -CFH-, or an alkylene group consisting of unit represented by -CFH- and a unit represented by -CH₂-, provided that these alkylene groups optionally contain epoxy group, -CH(OH)-, - CHI-, or a divalent cycloalkylene group;
   X is -OH, -CH(R²¹)OH (wherein R²¹ is H, a non-fluorinated alkyl group, or a fluorinated alkyl group), -I, -CFHI, -CH₂I, an anionic group, or -COOR²² (wherein R²² is a non-fluorinated alkyl group having 1 to 8 carbon atoms); and
   the total number of carbon atoms of R¹, R², and X is 2 to 50.
<2> According to the second aspect of the present disclosure, provided is the compound according to the first aspect, wherein R² is an alkylene group having 2 or more carbon atoms.
<3> According to the third aspect of the present disclosure, provided is the compound according to the first or second aspect, wherein R² is an alkylene group represented by general formula:

   - (CFH)ₙ₁-

   wherein n1 is an integer of 1 to 49.
<4> According to the fourth aspect of the present disclosure, provided is the compound according to the first or second aspect, wherein the compound is a fluoride of an unsaturated fatty acid.
<5> According to the fifth aspect of the present disclosure, provided is the compound according to any one of the first to fourth aspects, wherein the total number of carbon atoms of R¹, R², and X is 2 to 18.
<6> According to the sixth aspect of the present disclosure, provided is the compound according to any one of the first to fifth aspects, wherein the compound is a fluorine-containing alkyl iodide represented by general formula: R¹-CHF-(CHF-CHF)ₙ-I wherein R¹ is -CHF₂, -CHF₂, or -CHFI, and n is an integer of 0 or more.
<7> According to the seventh aspect of the present disclosure, provided is the compound according to any one of the first to fifth aspects, wherein the anionic group contains -COOM, -SO₃M, or -OSO₃M wherein M is a cation.
<8> According to the eighth aspect of the present disclosure, provided is a surfactant comprising at least one compound selected from the group consisting of compounds according to any of the first to fifth aspects wherein X in the general formula is -OH, -CH(R²¹)OH, or an anionic group.

### EXAMPLES

Next, embodiments of the present disclosure will now be described by way of Examples, but the present disclosure is not limited only to the Examples.

### Example 1 Synthesis of 1,1,2-trifluoro-2-iodoethane

To a 300 mL pressure resistant vessel, 37.1 g of iodine and 16.1 g of IF₅ were added, and the container was sealed. The container was cooled to -78°C, 10 g of (E)-1,2-difluoroethene was then introduced into the container, and the container was heated at 80°C for 20 hours. After the container was cooled with ice water, the content in the pressure resistant vessel was washed with water and then further washed with a 5% aqueous Na₂S₂O₄ solution to give 5.8 g of the title compound.
¹⁹F NMR (282MHz, CDCl₃): δ -169.1 - -169.4 (m, 1F), -124.0 - - 124.3 (m, 1F).
¹H NMR (400MHz, CDCl3): δ 6.79 (d with fine coupling, J = 48.0 Hz, 1H), 7.26 (td with fine coupling, J = 54.8, 3.6 Hz, 1H). LRMS (EI 70 eV) m/z (%): 210 (M+, 100), 190 (8), 171 (3), 83 (62), 64 (37), 51 (14).

### Example 2 Synthesis of 4,5,5-trifluoro-2-iodopentanol

To a 10 mL pressure resistant vessel, 1.84 g of 1,1,2-trifluoro-2-iodoethane, 509 mg of allyl alcohol, and 288 mg of azobisisobutyronitrile were added. Then, the container was heated at 80°C for 22 hours. After the container was cooled with ice water, the content in the pressure resistant vessel was analyzed by gas chromatography mass spectrometry, and it was found that the title compound was produced in an area ratio of 75.9% relative to an area ratio of 24.1% of 1,1,2-trifluoro-2-iodoethane as the raw material.

LRMS (EI 70 eV) m/z (%): 268 (M+, 1), 251 (1), 185 (2), 141 (95), 73 (100), 51 (38).

### Example 3 Synthesis of 7,8,8-trifluoro-5-iodooctan-1-ol

To a 10 mL pressure resistant vessel, 1.00 g of 1,1,2-trifluoro-2-iodoethane, 477 mg of 5-hexen-1-ol, and 235 mg of azobisisobutyronitrile were added. Then, the container was heated at 80°C for 22 hours. After the container was cooled with ice water, the content in the pressure resistant vessel was analyzed by gas chromatography mass spectrometry, and it was found that the title compound was produced in an area ratio of 66.8% (two isomers in total) relative to an area ratio of 37.2% of 1,1,2-trifluoro-2-iodoethane as the raw material.
LRMS (EI 70 eV) m/z (%): 293 ([M-OH]+, 100), 259 (5), 207 (25), 207 (25), 155 (34).

### Example 4 Synthesis of methyl 7,8,8-trifluoro-5-iodooctanoate

To a 10 mL pressure resistant vessel, 200 mg of 1,1,2-trifluoro-2-iodoethane, 122 mg of methyl 5-hexenoate, and 46.9 mg of azobisisobutyronitrile were added. Then, the container was heated at 80°C for 22 hours. After the container was cooled with ice water, the content in the pressure resistant vessel was analyzed by gas chromatography mass spectrometry, and it was found that the title compound was produced in an area ratio of 70.0% (two isomers in total) relative to an area ratio of 30.0% of 1,1,2-trifluoro-2-iodoethane as the raw material.

LRMS (EI 70eV) m/z (%): 307 ([M-OMe]+, 21), 211 (100), 192 (5), 151 (35).

### Example 5 Synthesis of 7,8,8-trifluoro-5-iodooctanoic acid

To a 10 mL pressure resistant vessel, 200 mg of 1,1,2-trifluoro-2-iodoethane, 109 mg of 5-hexenic acid, and 46.9 mg of azobisisobutyronitrile were added. Then, the container was heated at 80°C for 22 hours. After the container was cooled with ice water, a mixture containing the target compound was obtained. 20 mg of the mixture was taken in another glass container and diluted with 1 mL of tetrahydrofuran and 0.2 mL of methanol, trimethylsilyl diazomethane (a 10% hexane solution) was then added thereto, the mixture was stirred at room temperature, the content was then analyzed by gas chromatography mass spectrometry, and it was found that methyl 7,8,8-trifluoro-5-iodooctanoate obtained by methylation of the carboxylic acid of the title compound was produced in an area ratio of 85.7% (two isomers in total) relative to an area ratio of 14.3% of 1,1,2-trifluoro-2-iodoethane as the raw material.

LRMS (EI 70 eV) m/z (%): 307 ([M-OMe]+, 24), 211 (100), 192 (4), 151 (32).

### Example 6 Synthesis of 7,8,8-trifluorooctan-1-ol

To a 10 mL glass container, a mixed solution of 200 mg of 7,8,8-trifluoro-5-iodooctan-1-ol and 0.4 mL of methanol, and 67.5 mg of zinc were added. 0.33 mL of a 2 M aqueous hydrochloric acid solution was added. After the mixture was stirred for 6 hours, the content was analyzed by gas chromatography mass spectrometry, and it was found that the title compound was produced in an area ratio of 75.6% relative to an area ratio of 24.4% of 7,8,8-trifluoro-5-iodooctan-1-ol as the raw material.

LRMS (EI 70 eV) m/z (%): 167 ([M-OH]+, 100), 127 (54), 51 (4).

### Example 7 Oligomerization reaction of 1,1,2-trifluoro-2-iodoethane with (E)-1,2-difluoroethene

To a 30 mL pressure resistant vessel, 1.00 g of 1,1,2-trifluoro-2-iodoethane and 0.35 mL of 2-ethylhexanoyl(tert-butyl)peroxide were added, the container was sealed and cooled to -78°C, and 1.5 g of (E)-1,2-difluoroethene was then introduced. The container was heated at 80°C for 24 hours. Then, the container was cooled with ice water, the content in the pressure resistant vessel was then analyzed by gas chromatography mass spectrometry, and it was found that H-CF₂CHF-(CHFCHF)-I, H-CF₂CHF-(CHFCHF)₂-I, and H-CF₂CHF-(CHFCHF)₃-I were respectively produced in an area ratio of 36.1% (four isomers in total), 20.5% (eight isomers in total), and 21.3% (a plurality of isomers in total) relative to an area ratio of 22.2% of 1,1,2-trifluoro-2-iodoethane as the raw material. H-CF₂CHF-(CHFCHF)-I: LRMS (EI 70eV) m/z (%): 274 (M+, 87), 191 (11), 159 (30), 147 (76), 83 (45), 77 (100), 51 (82). H-CF₂CHF-(CHFCHF)₂-I: LRMS (EI 70eV) m/z (%): 338 (M+, 18), 211 (4), 191 (27), 159 (22), 147 (27), 83 (38), 77 (84), 51 (100). H-CF₂CHF-(CHFCHF)₃-I: LRMS (EI 70eV) m/z (%): 402 (M+, 2), 191 (23), 159 (34), 147 (18), 83 (36), 77 (89), 51 (100).

### Example 8

To a 500 mL pressure resistant vessel, 300 g of water, 0.31 g of Na₂HPO₄, and 0.48 g of ammonium persulfate were added, the container was sealed and cooled to -78°C, (E)-1,2-difluoroethene was then introduced, and the mixture was heated and reacted at 0.5 MPa and 90°C for 30 minutes. After being cooled, the resultant was sampled and dried at 50°C, and it was found that 0.77 wt% of solids were produced. The solids were analyzed by gel permeation chromatography (standard molecular weight of polystyrene), and it was found that an oligomer having Mn of 1,020 and Mw of 1,043 was obtained. As a result of the analysis by IR, almost no carbonyl stretching at 1,742 cm⁻¹ was observed. This implies that an oligomer having a sulfate group is contained as an anionic group. The surface tension of the obtained solids was measured and found to be 51.9 mN/m, and the obtained solids were demonstrated to have an efficacy as a surfactant.

## Claims

1. A compound represented by general formula:
R¹-R²-X
wherein
R¹ is -CH₃, -CH₂F, -CHF₂, -CH₂I, -CHFI, or an anionic group;
R² is an alkylene group consisting of unit represented by -CFH-, or an alkylene group consisting of unit represented by -CFH- and unit represented by -CH₂-, provided that these alkylene groups optionally contain epoxy group, -CH(OH)-, - CHI-, or a divalent cycloalkylene group;
X is -OH, -CH(R²¹)OH (wherein R²¹ is H, a non-fluorinated alkyl group, or a fluorinated alkyl group), -I, -CFHI, -CH₂I, an anionic group, or -COOR²² (wherein R²² is a non-fluorinated alkyl group having 1 to 8 carbon atoms); and
the total number of carbon atoms of R¹, R², and X is 2 to 50.

2. The compound according to claim 1, wherein R² is an alkylene group having 2 or more carbon atoms.

3. The compound according to claim 1 or 2, wherein R² is an alkylene group represented by general formula:
- (CFH)ₙ₁-
wherein n1 is an integer of 1 to 49.

4. The compound according to claim 1 or 2, wherein the compound is a fluoride of an unsaturated fatty acid.

5. The compound according to any one of claims 1 to 4, wherein the total number of carbon atoms of R¹, R², and X is 2 to 18.

6. The compound according to any one of claims 1 to 5, wherein the compound is a fluorine-containing alkyl iodide represented by general formula: R¹-CHF-(CHF-CHF)ₙ-I wherein R¹ is -CHF₂, -CHF₂, or -CHFI, and n is an integer of 0 or more.

7. The compound according to any one of claims 1 to 5, wherein the anionic group contains -COOM, -SO₃M, or -OSO₃M wherein M is a cation.

8. A surfactant comprising at least one compound selected from the group consisting of compounds according to any of claims 1 to 5 wherein X in the general formula is -OH, -CH(R²¹)OH, or an anionic group.
